# EUROPÄISCHE PATENTSCHRIFT

(11) **EP 3 694 592 B1**
(45) Veröffentlichungstag und Bekanntmachung des Hinweises auf die Patenterteilung: **23.11.2022**
(21) Anmeldenummer: 18782933.8
(22) Anmeldetag: 01.10.2018
(51) Int. Cl.: A61M 16/20, F04B 45/047, A61M 16/00, A61M 16/04

(54) **VORRICHTUNG ZUM BEATMEN EINES PATIENTEN**
DEVICE FOR VENTILATING A PATIENT
DISPOSITIF POUR ASSURER LA RESPIRATION ARTIFICIELLE D'UN PATIENT

(30) Priorität: 13.10.2017 DE 102017009603
(43) Veröffentlichungstag der Anmeldung: 19.08.2020
(73) Patentinhaber: Drägerwerk AG & Co. KGaA, 23558 Lübeck (DE)
(72) Erfinder: HANSMANN, Hans-Ullrich, 23858 Barnitz (DE); HILTAWSKY, Karsten, 23617 Stockelsdorf (DE)
(86) Internationale Anmeldenummer: PCT/EP2018/076566
(87) Internationale Veröffentlichungsnummer: WO 2019/072606

(56) Entgegenhaltungen:
- WO-A1-2018/033225
- US-A- 5 063 925
- US-A1- 2013 167 843
- US-A1- 2013 331 715

## Beschreibung

Die Erfindung betrifft eine zur Verwendung zusammen mit einer Druckquelle, wie vorzugsweise einer Konstantdruckquelle oder einem Beatmungsgerät, vorgesehene und im Folgenden als Patientenmodul bezeichnete Vorrichtung. Eine Konstantdruckquelle ist beispielsweise eine Gasflasche, eine Gasfördereinheit zur Bereitstellung eines konstanten Gasdrucks oder eine Gasversorgungseinheit wie z.B. eine Wandversorgungseinheit in einem Krankenhaus.

Als Vorrichtungen zum Beatmen eines Patienten sind z. B. Beatmungsgeräte oder Anästhesiegeräte bekannt. Beatmungsgeräte und Anästhesiegeräte - im Folgenden zusammenfassend als Beatmungsgeräte oder einzeln als Beatmungsgerät bezeichnet - werden dazu genutzt, Patienten, die entweder gar nicht selbständig atmen können oder Hilfe beim Atmen benötigen, Atemluft bereitzustellen. Dazu tragen die Patienten z.B. eine Gesichtsmaske, welche Mund und Nase abdeckt, oder einen Tubus, der in den Rachenraum und die Luftröhre des Patienten eingeführt ist. Die Gesichtsmaske oder der Tubus - im Folgenden zusammenfassend als Patientenschnittstelle bezeichnet - sind über zumindest einen Beatmungsschlauch mit dem Beatmungsgerät verbunden. Die Patientenschnittstelle kann auch eine Trachealkanüle sein.

Eine Aufgabe der vorliegenden Erfindung besteht darin, eine leicht auswechselbare Vorrichtung zum Beatmen eines Patienten anzugeben.

Erfindungsgemäß wird diese Aufgabe mittels einer im Folgenden als Patientenmodul bezeichneten, räumlich von einer Druckquelle, insbesondere einem als Druckquelle fungierenden Beatmungsgerät, getrennten und mit der Druckquelle pneumatisch verbundenen Vorrichtung zum Beatmen eines Patienten mit den Merkmalen des Anspruchs 1 gelöst.

Dazu ist bei einem derartigen, zur Verwendung zusammen mit einer Druckquelle bestimmten Patientenmodul vorgesehen, dass das Patientenmodul die Druckquelle strömungsmäßig an eine mit den Atemwegen eines Patienten verbundene oder verbindbare Patientenschnittstelle koppelt und dass das Patientenmodul zumindest eine als Exspirationsventil fungierende Ventilanordnung umfasst. Bei zwei Ventilanordnungen fungiert eine Ventilanordnung als Exspirationsventil und die andere Ventilanordnung als Inspirationsventil.

Bezüglich der oder jeder Ventilanordnung ist insbesondere Folgendes vorgesehen: Die oder jede Ventilanordnung umfasst einen Ventilantrieb, eine Druckkammer und eine Steuerdruckkammer. Der Ventilantrieb ist zum Erzeugen eines Steuerdrucks in der Steuerdruckkammer fluidkommunizierend mit der Steuerdruckkammer verbunden und als Ventilantrieb fungiert eine Piezopumpe, welche vorzugsweise hochfrequent betreibbar ist. Die Steuerdruckkammer ist von der Druckkammer mittels eines ein Verschlusselement aufweisenden Membranelements getrennt. Mittels des Verschlusselements kann eine erste Öffnung der Druckkammer geöffnet und verschlossen werden. Mittels des auf das erste Membranelement einwirkenden Steuerdrucks ist das Verschlusselement, nämlich eine Position des Verschlusselements, steuerbar.

Der Vorteil des gegenständlichen Patientenmoduls besteht darin, dass wichtige Funktionselemente nahe zum Patienten, nämlich in das Patientenmodul oder zumindest in die Umgebung des Patientenmoduls, verlagert sind und dass das Patientenmodul leicht und unkompliziert zum Beispiel zu Reinigungszwecken gegen ein anderes Patientenmodul ausgetauscht werden kann. Das Patientenmodul kann zwischen die jeweilige Druckquelle und eine jeweilige Patientenschnittstelle gekoppelt werden und bildet in einer die Druckquelle, das Patientenmodul und die Patientenschnittstelle umfassenden Wirkkette ein austauschbares, lösbar einerseits mit der Druckquelle und andererseits mit der Patientenschnittstelle verbindbares Modul.

Indem das Patientenmodul zumindest eine als Exspirationsventil fungierende Ventilanordnung umfasst, kann eine sehr einfache Druckquelle bzw. Konstantdruckquelle verwendet werden, nämlich zum Beispiel eine Druckgasflasche, welche über einen Beatmungsschlauch zwischen der Druckquelle und dem Patientenmodul einen Volumenstrom mit Atemgas und einen Überdruck gegenüber dem Umgebungsdruck zur Verfügung stellt. Mittels dieses Volumenstroms kann in grundsätzlich an sich bekannter Art und Weise während der inspiratorischen Phase die Beatmung des Patienten erfolgen. Ein Druckverlauf während der inspiratorischen Phase wird durch Ansteuerung des Exspirationsventils gesteuert oder geregelt. Während einer an die inspiratorische Phase anschließenden exspiratorischen Phase wird das Exspirationsventil in ebenfalls grundsätzlich an sich bekannter Art und Weise zum Erhalt eines für die Exspiration notwendigen Druckunterschieds zum Druck in der Patientenlunge gesteuert oder geregelt geöffnet.

Ein Patientenmodul mit einem Inspirationsventil, einem Exspirationsventil und als Ventilantriebe fungierenden Piezopumpen ist aus der US 2013/167843 A1 bekannt. Aus der nicht vorveröffentlichten WO 2018/033225 A1 ist eine pneumatische Steuervorrichtung bekannt, die einfach aufgebaut ist und eine schnelle Steuerung von pneumatischen Steuerungselementen ermöglicht.

Vorteilhafte Ausgestaltungen der Erfindung sind Gegenstand der Unteransprüche. Dabei verwendete Rückbeziehungen innerhalb der Ansprüche weisen auf die weitere Ausbildung des Gegenstandes des in Bezug genommenen Anspruchs durch die Merkmale des jeweiligen abhängigen Anspruchs hin. Sie sind nicht als ein Verzicht auf die Erzielung eines selbständigen, gegenständlichen Schutzes für die Merkmale oder Merkmalskombinationen eines abhängigen Anspruchs zu verstehen. Des Weiteren ist im Hinblick auf eine Auslegung der Ansprüche sowie der Beschreibung bei einer näheren Konkretisierung eines Merkmals in einem abhängigen Anspruch davon auszugehen, dass eine derartige Beschränkung in den jeweils vorangehenden Ansprüchen sowie einer allgemeineren Ausführungsform des gegenständlichen Patientenmoduls nicht vorhanden ist. Jede Bezugnahme in der Beschreibung auf Aspekte abhängiger Ansprüche ist demnach auch ohne speziellen Hinweis ausdrücklich als Beschreibung optionaler Merkmale zu lesen.

Bei einer Ausführungsform des Patientenmoduls ist vorgesehen, dass der oder jeder Ventilantrieb ein mit einer elektrischen Spannung beaufschlagbares Piezoelement umfasst und dass mittels des Piezoelements durch eine spannungsabhängige Formänderung des Piezoelements ein Pumpenmembranelement des Ventilantriebs beweglich ist. Die Beweglichkeit des Pumpenmembranelements begründet die Funktion des Ventilantriebs als Antrieb der Ventilanordnung. Der Ventilantrieb tritt innerhalb der Ventilanordnung an die Stelle eines bisher erforderlichen mechanischen Aktors. Durch die Verwendung eines Piezoelements kann die Ventilanordnung sehr klein (miniaturisiert) ausgeführt sein und das Pumpenmembranelement kann vorzugsweise mit einer hohen Frequenz bewegt werden.

Bei einer besonderen Ausführungsform des Patientenmoduls ist der Ventilantrieb der zumindest einen Ventilanordnung des Patientenmoduls innerhalb oder außerhalb des Patientenmoduls anordenbar. Bei einem innerhalb des Patientenmoduls angeordneten Ventilantrieb ergibt sich eine kompakte, einteilige Bauform. Bei einem außerhalb des Patientenmoduls angeordneten Ventilantrieb sind ein Austausch und eine eventuelle Entsorgung des Patientenmoduls möglich und der Ventilantrieb bleibt für eine eventuelle spätere Verwendung an einem anderen Patientenmodul erhalten.

Bei einer Ausführungsform des Patientenmoduls weist dieses Mittel zum lösbaren Verbinden mit der Patientenschnittstelle und/oder Mittel zum lösbaren Verbinden mit zumindest einem von der Druckquelle kommenden Beatmungsschlauch auf.

Die lösbare Verbindbarkeit des Patientenmoduls mit der Patientenschnittstelle und/oder mit dem zumindest einen von der Druckquelle kommenden Beatmungsschlauch gewährleistet eine leichte Austauschbarkeit eines Patientenmoduls gegen ein anderes Patientenmodul. Aufgrund der lösbaren Verbindbarkeit ist für einen solchen Austausch kein Techniker notwendig. Vielmehr kann ein solcher Austausch ohne Weiteres auch von Krankenhauspersonal oder sogar vom Verwender des Patientenmoduls vorgenommen werden.

Besonders einfach ist das Lösen und spätere Wiederherstellen einer solchen lösbaren Verbindung, wenn dafür ein Medizinkonus verwendet wird. Auf einer Eingangsseite des Patientenmoduls wird der oder jeder Beatmungsschlauch zum Austausch des Patientenmoduls am jeweiligen Medizinkonus abgezogen und später auf den eingangsseitigen Medizinkonus eines neuen Patientenmoduls aufgesteckt. Ebenso wird auf einer Ausgangsseite des Patientenmoduls die Patientenschnittstelle oder ein zur Patientenschnittstelle führendes Schlauchstück vom dortigen Medizinkonus gelöst und später auf den ausgangsseitigen Medizinkonus des neuen Patientenmoduls aufgesteckt.

Bei einer weiteren, zusätzlichen oder alternativen Ausführungsform des Patientenmoduls weist dieses Mittel zum lösbaren Verbinden mit zumindest einem außerhalb des Patientenmoduls angeordneten Ventilantrieb auf. Ein Schlauch zwischen dem Ventilantrieb und den restlichen Einheiten der Ventilanordnung, welcher an zumindest einer Verbindungsstelle entweder von dem Ventilantrieb oder den sonstigen Einheiten der Ventilanordnung lösbar ist, ist ein Beispiel für ein Mittel zum lösbaren Verbinden des zumindest einen Ventilantriebs mit dem Patientenmodul, nämlich mit den im Innern des Patientenmoduls befindlichen Einheiten der Ventilanordnung.

Bei einer speziellen Ausführungsform umfasst das Patientenmodul zwei Ventilanordnungen mit jeweils innerhalb oder außerhalb des Patientenmoduls anordenbaren Ventilantrieben, wobei eine der zumindest zwei Ventilanordnungen als Exspirationsventil und eine der Ventilanordnungen als Inspirationsventil fungiert. Bei einem Patientenmodul, welches zumindest eine als Exspirationsventil sowie eine als Inspirationsventil fungierende Ventilanordnung umfasst, kann ein jeweiliger Druck- und/oder Volumenstromverlauf während der inspiratorischen und exspiratorischen Phasen mittels des Inspirationsventils und des Exspirationsventils patientennah, nämlich mittels des selbst patientennah angeordneten Patientenmoduls und der davon umfassten Ventilanordnungen, gesteuert und/oder geregelt werden.

Eine nochmals weitere Ausführungsform des Patientenmoduls zeichnet sich durch eine vom Patientenmodul umfasste Sensorik sowie eine gegebenenfalls auch entfernt von dem Patientenmodul anordenbare Steuerungseinrichtung aus, wobei mittels der Steuerungseinrichtung auf Basis zumindest eines von der Sensorik erhältlichen Sensorsignals zumindest ein Steuersignal zur Ansteuerung der zumindest einen Ventilanordnung generierbar ist. Der Vorteil der von dem Patientenmodul umfassten und damit patientennah angeordneten Sensorik besteht vor allem darin, dass diese für eine exakte Ansteuerung der zumindest einen Ventilanordnung besonders gut verwendbare Sensorsignale liefert. Die Qualität der Sensorsignale ist vor allem gegenüber Sensorsignalen von einer Sensorik im Bereich eines als Druckquelle fungierenden Beatmungsgeräts verbessert. Ein Sensorsignal von einer Sensorik im oder am Beatmungsgerät repräsentiert immer einen Zustand, welcher gegenüber einem aktuellen Zustand im Bereich einer Atemmaske oder dergleichen aufgrund der Laufzeit der pneumatischen Verhältnisse entlang des zumindest einen Beatmungsschlauchs zum Beatmungsgerät gewissermaßen bereits veraltet ist. Darüber hinaus wirkt der zumindest eine Beatmungsschlauch zum Beatmungsgerät wie ein Tiefpassfilter, so dass ein von einer Sensorik im oder am Beatmungsgerät erhältliches Sensorsignal bereits hinsichtlich seiner Dynamik reduziert ist.

Ein Patientenmodul, welches eine solche Sensorik und zusätzlich zumindest eine als Exspirationsventil fungierende Ventilanordnung oder eine als Exspirationsventil fungierende Ventilanordnung sowie eine als Inspirationsventil fungierende Ventilanordnung umfasst, umfasst die wesentlichen Funktionseinheiten für eine Beatmung eines Patienten, welche bisher in einem im Vergleich zum Patientenmodul patientenfern aufgestellten Beatmungsgerät enthalten waren.

Bei einer besonderen Ausführungsform eines solchen Patientenmoduls ist die Steuerungseinrichtung vom Patientenmodul räumlich getrennt und zum Erhalt des zumindest einen Sensorsignals von der Sensorik sowie zur Übermittlung des zumindest einen Steuersignals an die zumindest eine Ventilanordnung des Patientenmoduls mit dem Patientenmodul kommunikativ verbindbar und beim Betrieb des Patientenmoduls kommunikativ mit diesem verbunden. Der Vorteil dieser Ausführungsform besteht vor allem darin, dass bei einem eventuellen Austausch des Patientenmoduls auch die Steuerungseinrichtung erhalten bleiben kann und für eine spätere Verwendung mit einem anderen Patientenmodul zur Verfügung steht. Die Steuerungseinrichtung kann dabei im Vergleich zu dem patientennahen Patientenmodul durchaus patientenfern angeordnet sein, so dass der Patient, der das Patientenmodul zum Beispiel am Körper tragen kann, nicht ebenfalls die Steuerungseinrichtung am Körper tragen muss. Genauso kann vorgesehen sein, dass auch die Steuerungseinrichtung am Körper getragen werden kann. Ebenfalls kann vorgesehen sein, dass die Steuerungseinrichtung in oder an einem als Druckquelle fungierenden Beatmungsgerät angeordnet ist oder Teil der Funktionalität des Beatmungsgeräts ist. Im Letzteren Fall ergibt sich ein besonderer Vorteil dadurch, dass dann das Beatmungsgerät als Bedien- und Benutzerschnittstelle für das Patientenmodul fungiert und Bedien- und/oder Anzeigeelemente, welche am Beatmungsgerät ohnehin vorhanden sind, auch zum Bedienen, insbesondere Parametrieren, des Patientenmoduls verwendbar sind und dass mittels des als Bedien- und Benutzerschnittstelle für das Patientenmodul fungierenden Beatmungsgeräts eine Funktionskontrolle des Patientenmoduls möglich ist.

Der Betrieb des Patientenmoduls erfolgt automatisch unter Kontrolle der Steuerungseinrichtung. Diese umfasst dafür in grundsätzlich an sich bekannter Art und Weise eine Verarbeitungseinheit in Form von oder nach Art eines Mikroprozessors sowie einen Speicher. In den Speicher ist ein von der Verarbeitungseinheit ausführbares Steuerungsprogramm geladen oder ladbar, das beim Betrieb des Patientenmoduls durch dessen Verarbeitungseinheit ausgeführt wird. Bedienhandlungen eines Benutzers können entweder an der Steuerungseinrichtung oder an dem dann als Bedien- und Benutzerschnittstelle der Steuerungseinrichtung und des Patientenmoduls fungierenden Beatmungsgerät vorgenommen werden.

Soweit das mittels der Steuerungseinrichtung ausführbare und beim Betrieb des Patientenmoduls ausgeführte Steuerungsprogramm betroffen ist, fungieren die Steuerungseinrichtung und das Steuerungsprogramm als Mittel zur Durchführung eines Verfahrens zum Betrieb des Patientenmoduls. Die Erfindung ist damit einerseits auch ein Computerprogramm mit durch einen Computer ausführbaren und von dem Steuerungsprogramm umfassten Programmcodeanweisungen und andererseits ein Speichermedium mit einem derartigen Computerprogramm, also ein Computerprogrammprodukt mit Programmcodemitteln, sowie schließlich auch eine Steuerungseinrichtung, in deren Speicher als Mittel zur Durchführung des Verfahrens zum Betrieb des Patientenmoduls ein solches Computerprogramm geladen oder ladbar ist.

Insgesamt ist die Erfindung auch ein Patientenmodulsystem mit einem Patientenmodul sowie mit einer innerhalb oder außerhalb des Patientenmoduls anordenbaren Steuerungseinrichtung wie jeweils hier und im Folgenden beschrieben, wobei das Patientenmodul ferner eine Sensorik umfasst und wobei mittels der Steuerungseinrichtung auf Basis zumindest eines von der Sensorik erhältlichen Sensorsignals zumindest ein Steuersignal zur Ansteuerung zumindest einer Ventilanordnung des Patientenmoduls generierbar ist und im Betrieb zur Beatmung des Patienten generiert wird. Bevorzugt kann die Steuerungseinrichtung vom Patientenmodul räumlich getrennt sein. Die Steuerungseinrichtung ist dann zum Erhalt des zumindest einen Sensorsignals von der Sensorik sowie zur Übermittlung des zumindest einen Steuersignals an zumindest eine Ventilanordnung des Patientenmoduls mit dem Patientenmodul kommunikativ verbunden.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen.

Das Ausführungsbeispiel ist nicht als Einschränkung der Erfindung zu verstehen. Vielmehr sind im Rahmen der vorliegenden Offenbarung Abänderungen und Modifikationen möglich, insbesondere solche Varianten und Kombinationen, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen für den Fachmann im Hinblick auf die Lösung der Aufgabe entnehmbar sind und durch kombinierbare Merkmale zu einem neuen Gegenstand führen.

Es zeigen:
- Figur 1: eine Patientenlunge, eine zur Beatmung des Patienten vorgesehene Druckquelle, ein nahe am Patienten befindliches Patientenmodul und eine Patientenschnittstelle,
- Figur 2: das Patientenmodul mit weiteren Einzelheiten, nämlich zumindest einer von dem Patientenmodul umfassten Ventilanordnung,
- Figur 3: eine Ausführungsform einer von dem Patientenmodul umfassten Ventilanordnung,
- Figur 4: eine Ausführungsform eines Ventilantriebs einer Ventilanordnung,
- Figur 5: eine alternative Ausführungsform einer Ventilanordnung gemäß Figur 3,
- Figur 6: eine weitere alternative Ausführungsform einer Ventilanordnung gemäß Figur 3 mit einem räumlich von den sonstigen Elementen der Ventilanordnung entfernt anordenbaren Ventilantrieb,
- Figur 7: ein Membranelement und ein dem Membranelement zugeordnetes Verschlusselement sowie die auf die unterschiedlichen Seiten (Unter- oder Oberseite) und Abschnitte (Membranelement oder Verschlusselement) wirkenden Drücke,
- Figur 8: eine Steuerungseinrichtung zum Betrieb des Patientenmoduls und
- Figur 9: das Patientenmodul gemäß Figur 2 mit einer Veranschaulichung möglicher Orte von Ventilantrieben zu der oder jeder vom Patientenmodul umfassten Ventilanordnung.

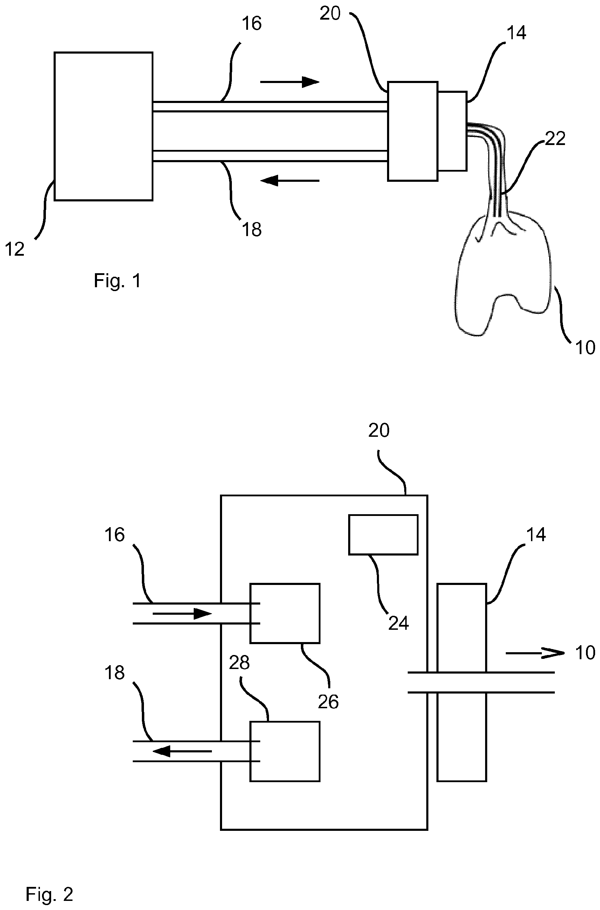

Die Darstellung in Figur 1 zeigt - schematisch stark vereinfacht - die Lunge eines Patienten (Patientenlunge 10) und eine Druckquelle 12. Die Druckquelle 12 ist vorzugsweise eine Konstantdruckquelle, beispielsweise eine Gasflasche, eine Gasfördereinheit zur Bereitstellung eines konstanten Gasdrucks oder eine Gasversorgungseinheit. Als Druckquelle 12 fungiert vorzugsweise ein zur Beatmung des Patienten vorgesehenes Medizingerät, insbesondere ein Medizingerät in Form eines Beatmungsgeräts mit einer Druckquelle 12 oder eines Beatmungsgeräts mit einer Druckquelle in Form eines kombinierten Anästhesie- und Beatmungsgeräts. Genauso kommen als Druckquelle 12 eine Druckgasflasche, ein Verdichter, insbesondere ein Radialverdichter, oder ein Wandauslass eines medizinischen Druckluftsystems, zum Beispiel in einem Krankenhaus, in Betracht. Die Druckquelle 12 kann also vorzugsweise eine Konstantdruckquelle in Form einer Druckgasflasche, eines Verdichters, insbesondere eines Radialverdichters, oder ein Wandauslasses eines medizinischen Druckluftsystems, zum Beispiel in einem Krankenhaus, sein.

Die Druckquelle 12 ist mittelbar mittels eines im Folgenden als Patientenschnittstelle 14 bezeichneten Kopplungsstücks sowie mittels zumindest eines Beatmungsschlauchs 16, 18, nämlich mittels eines Inspirationsschlauchs 16 oder mittels eines Inspirationsschlauchs 16 und eines Exspirationsschlauchs 18, mit den Atemwegen des Patienten und der Patientenlunge 10 verbunden. Die Pfeile oberhalb des Inspirationsschlauchs 16 und unterhalb des Exspirationsschlauchs 18 veranschaulichen die im Betrieb resultierende Richtung des Volumenstroms. Anstelle eines an die Druckquelle 12 angeschlossenen Exspirationsschlauchs 18 kann dieser zum Beispiel in Form eines kurzen Schlauchstücks auch einseitig zum Umgebungsdruck offen sein.

Nahe am Patienten, zum Beispiel vom Patienten selbst getragen, befindet sich ein Patientenmodul 20, an welches der zumindest eine Beatmungsschlauch 16, 18 angeschlossen ist. Optional umfasst das Patientenmodul 20 intern ein sogenanntes Y-Stück, mit welchem der Inspirationszweig und der Exspirationszweig in grundsätzlich an sich bekannter Art und Weise zusammengefasst und in zusammengefasster Form auf die Patientenschnittstelle 14 geführt sind. Ohne ein solches Y-Stück fungiert das Patientenmodul 20 und ein das Patientenmodul 20 umgebendes Gehäuse selbst als Mittel zur Zusammenführung des Inspirationszweigs und des Exspirationszweigs auf die Patientenschnittstelle 14.

Bei der Patientenschnittstelle 14 kann es sich um eine zur Beatmung eines Patienten vorgesehene sogenannte Gesichtsmaske oder dergleichen handeln. Darüber hinaus kann es sich bei der Patientenschnittstelle 14 auch um einen sogenannten Tubus 22 (Endotrachealtubus) oder eine Endotrachealkanüle handeln. Das oben erwähnte Y-Stück kann sich im oder am Patientenmodul 20 befinden oder Teil der Patientenschnittstelle 14 sein.

Die Darstellung in Figur 2 zeigt - weiterhin schematisch stark vereinfacht - eine Ausführungsform des Patientenmoduls 20 mit weiteren Einzelheiten. Danach umfasst das Patientenmodul 20 eine ggf. auch verteilte Sensorik 24, welche zumindest einen Sensor, zum Beispiel einen Drucksensor und/oder einen Flowsensor, umfasst. Des Weiteren sind bei der gezeigten Ausführungsform - jeweils im Patientenmodul 20 - dem Ende des Inspirationsschlauchs 16 und dem Ende des Exspirationsschlauchs 18 jeweils ein Ventil 26, 28 (Inspirationsventil 26, Exspirationsventil 28) zugeordnet. Beide Ventile 26, 28 sind vorzugsweise gleich oder zumindest gleichartig ausgeführt und die nachfolgend erörterten Darstellungen befassen sich mit möglichen Ausführungsformen dieser Ventile 26, 28. Das Inspirationsventil 26 ist grundsätzlich optional. Bei einer Ausführungsform ohne Inspirationsventil 26 wird der inspiratorische Druck und dessen Änderung während der inspiratorischen Phase durch eine Ansteuerung des Exspirationsventils 28 eingestellt und dabei zum Erhalt der gewünschten Druckverlaufs zum Beispiel das Patientenmodul 20 gewissermaßen gesteuert zur Umgebung geöffnet.

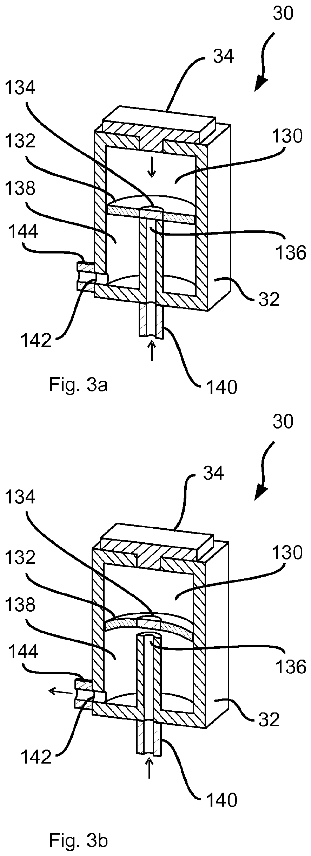

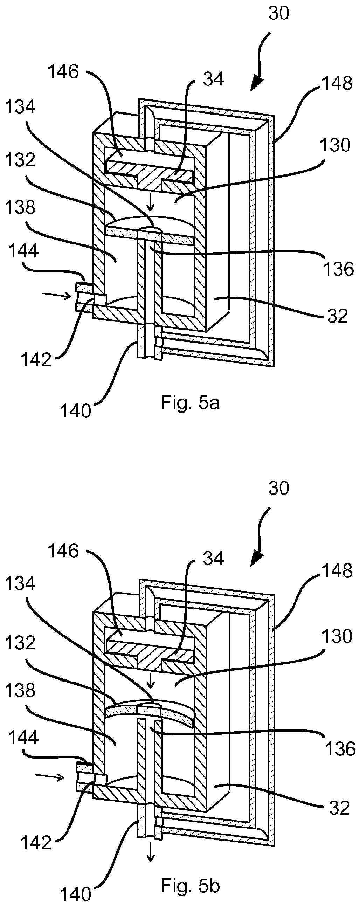

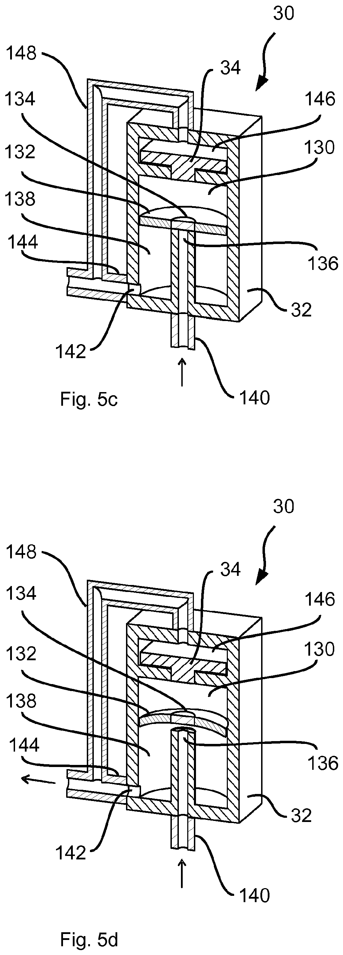

Die Darstellungen in Figur 3 (Figuren 3a, 3b) und Figur 5 (Figuren 5a bis 5d) zeigen eine als pneumatische Steuervorrichtung fungierende Ventilanordnung 30. Die Ventilanordnung 30 gemäß Figur 3 kommt als Inspirationsventil 26 und als Exspirationsventil 28 in Betracht. Die Ventilanordnung 30 gemäß Figur 5 kommt ebenfalls als Inspirationsventil 26 in Betracht. Jede Ventilanordnung 30 umfasst ein Gehäuse 32 und eine mit dem Gehäuse 32 verbundene und als Ventilantrieb 34 fungierende Pumpvorrichtung. Als Pumpvorrichtung / Ventilantrieb 34 fungiert eine Piezopumpe. Die Pumpvorrichtung / die Piezopumpe ist vorzugsweise in zwei Richtungen durchströmbar und ist somit eine Zwei-Wege-Pumpe.

Eine Ventilanordnung 30 kann mehr als einen Ventilantrieb 34 (Pumpvorrichtung / Piezopumpe) aufweisen. Die Piezopumpen können dabei als Stack aus in Reihe geschalteten Piezopumpen ausgebildet sein. Mittels der Stackbildung können die Pumpendrücke mehrerer Piezopumpen zusammengefasst werden. Alternativ können mehrere parallel geschaltete Piezopumpen in der Ventilanordnung 30 vorhanden sein.

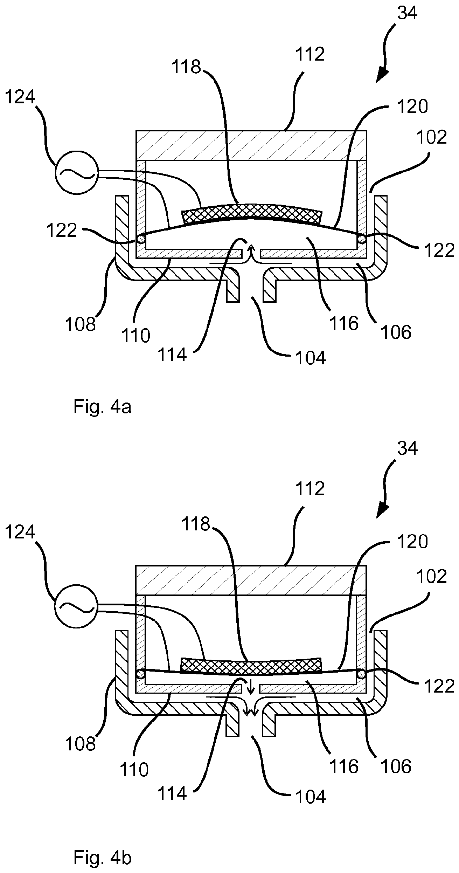

Figur 4 (Figuren 4a, 4b) zeigt den Ventilantrieb 34 mit weiteren Details. Danach weist der Ventilantrieb 34 eine erste Zwei-Wege-Durchlassöffnung 102 und eine zweite Zwei-Wege-Durchlassöffnung 104 auf, die durch einen Zwei-Wege-Kanal 106 verbunden sind. Der Zwei-Wege-Kanal 106 verläuft zwischen einem Außengehäuse 108 und einem Innengehäuse 110. Die zweite Zwei-Wege-Durchlassöffnung 104 ist im Außengehäuse 108 gebildet. Die erste Zwei-Wege-Durchlassöffnung 102 ergibt sich aufgrund eines Abstands zwischen einem Rand des Außengehäuses 108 und dem angrenzenden Innengehäuse 110. Das Innengehäuse 110 ist mittels einer Abdeckplatte 112 verschlossen.

In dem Zwei-Wege-Kanal 106 ist im Innengehäuse 110 eine Pumpöffnung 114 angeordnet, die den Zwei-Wege-Kanal 106 mit einer Pumpenkammer 116 verbindet. In der Pumpenkammer 116 sind ein Piezoelement 118 und ein Pumpenmembranelement 120 angeordnet. Das Pumpenmembranelement 120 ist einerseits mit dem Piezoelement 118 und andererseits - über flexible Verbindungselemente 122 - mit dem Innengehäuse 110 verbunden. Das Piezoelement 118 wird mittels eines Wechselspannungsgenerators 124 in grundsätzlich an sich bekannter Art und Weise mit alternierenden elektrischen Spannungen beaufschlagt. Diese bewirken eine spannungsinduzierte Verformung des Piezoelements 118 und diese Verformung führt zu einer gesteuerten Schwingung des Pumpenmembranelements 120. Aufgrund einer mittels des Wechselspannungsgenerators 124 abgegebenen Wechselspannung, welche vorzugsweise hochfrequent ist, schwingt das Pumpenmembranelement 120 in der Pumpenkammer 116 mit entsprechender ,vorzugsweise hoher, Frequenz und im Ergebnis werden Pumpstöße durch die resultierende Volumenänderung der Pumpenkammer 116 erzeugt (Funktion der als Ventilantrieb 34 fungierenden Piezopumpe, vorzugsweise als Hochfrequenzpumpe). Diese Pumpstöße können durch die Pumpöffnung 114 in den Zwei-Wege-Kanal 106 hinein wirken und bewirken eine Strömung eines jeweiligen Fluids, zum Beispiel Luft, durch die zweite Zwei-Wege-Durchlassöffnung 104.

Die Strömung durch die Pumpöffnung 114, die aus der Pumpenkammer 116 hinaus gerichtet ist, ist auf die zweite Zwei-Wege-Durchlassöffnung 104 gerichtet. D.h., dass ein Pumpstoß, der durch eine Verkleinerung des Volumens der Pumpenkammer 116 erzeugt wird, durch die Pumpöffnung 114 direkt auf die zweite Zwei-Wege-Durchlassöffnung 104 gerichtet wird. In diesem Fall reißt die Strömung zwischen der Pumpöffnung 114 und der zweiten Zwei-Wege-Durchlassöffnung 104 das Fluid im Zwei-Wege-Kanal 106 mit, sodass eine Strömung von der ersten Zwei-Wege-Durchlassöffnung 102 zur zweiten Zwei-Wege-Durchlassöffnung 104 erzeugt wird. Bei einer Vergrößerung des Volumens der Pumpenkammer 116 wird das Fluid von dem Zwei-Wege-Kanal 106 durch die Pumpöffnung 114 in die Pumpenkammer 116 gesogen. In diesem Fall wird das Fluid aus dem Zwei-Wege-Kanal 106 in die Pumpenkammer 116 gesogen.

Die Pumpöffnung 114 ist dabei so weit von der zweiten Zwei-Wege-Durchlassöffnung 104 entfernt angeordnet, dass nur ein geringer Anteil an Fluid durch die zweite Zwei-Wege-Durchlassöffnung 104 in den Zwei-Wege-Kanal 106 durch die Pumpöffnung 114 in die Pumpenkammer 116 fließt. Der größere Teil des Fluids wird aus der ersten Zwei-Wege-Durchlassöffnung 102 durch den Zwei-Wege-Kanal 106 und die Pumpöffnung 114 in die Pumpenkammer 116 gesaugt. Wenn der Ventilantrieb (Piezopumpe) 34 ausgeschaltet ist, ist in dem Zwei-Wege-Kanal 106 keine gerichtete Strömung vorhanden. Vielmehr besteht dann zwischen der ersten Zwei-Wege-Durchlassöffnung 102 und der zweiten Zwei-Wege-Durchlassöffnung 104 ein freier Strömungsweg durch den Zwei-Wege-Kanal 106, der in beide Richtungen gerichtet sein kann. Es kann somit zwischen der ersten Zwei-Wege-Durchlassöffnung 102 und der zweiten Zwei-Wege-Durchlassöffnung 104 ein Druckausgleich stattfinden. Daher wird kein Entlastungsventil oder dergleichen benötigt.

Bei der Ausführungsform der Ventilanordnung 30 gemäß Figur 3a ist der Ventilantrieb 34 ausgebildet und angeordnet, um Luft von der Umgebung in eine Steuerdruckkammer 130 im Innern des Gehäuses 32 der Ventilanordnung 30 zu pumpen. Folglich wird mittels des Ventilantriebs 34 eine Druckerhöhung in der Steuerdruckkammer 130 erzeugt. Der unterhalb des Ventilantriebs 34 vertikal nach unten weisende Pfeil zeigt dabei die Steuerflussrichtung an, mit der eine Fluidströmung vom Ventilantrieb 34 weg (aus der Piezopumpe heraus) dargestellt wird. Der Ventilantrieb 34 ist mit der Steuerdruckkammer 130 fluidkommunizierend zum Erzeugen eines Steuerdrucks in der Steuerdruckkammer 130 verbunden.

Ein Membranelement 132 bildet zusammen mit einem Verschlusselement 134 eine elastisch bewegliche Wand der Steuerdruckkammer 130. Das Membranelement 132 ist weiter mit dem Verschlusselement 134 verbunden, insbesondere einstückig mit dem Verschlusselement 134 verbunden. Das Verschlusselement 134 ist dazu ausgebildet, eine erste Öffnung 136 einer im Innern des Gehäuses 32 gebildeten Druckkammer 138 zu verschließen oder zu öffnen. Das Membranelement 132 und das Verschlusselement 134 können vorzugsweise einstückig miteinander verbunden sein. Das Membranelement 132 und das Verschlusselement 134 unterteilen das Innere des Gehäuses 32 der Ventilanordnung 30 und grenzen die Steuerdruckkammer 130 von der Druckkammer 138 ab. Die erste Öffnung 136 kann einen Durchmesser von 1 mm bis 10 mm haben. Der gewählte Durchmesser der ersten Öffnung 136 hängt von dem Vordruck ab, mit dem die pneumatische Ventilanordnung 30 arbeitet.

Bei der in Figur 3a gezeigten Situation wird aufgrund eines erhöhten Drucks in der Steuerdruckkammer 130 das Membranelement 132 hin zu der Öffnung 136 ausgelenkt. Dabei wird das Verschlusselement 134 auf die erste Öffnung 136 gedrückt und die erste Öffnung 136 wird verschlossen. Bei der in Figur 3b gezeigten Situation ist das Membranelement 132 in entgegengesetzter Richtung ausgelenkt und die erste Öffnung 136 ist offen.

Wenn die Ventilanordnung 30 gemäß Figur 3 als Inspirationsventil 26 in einem Patientenmodul 20 (Fig. 2) fungiert, ist zum Beispiel an ein erstes Anschlussleitungselement 140, an dessen Ende sich im Innern des Gehäuses 32 die erste Öffnung 136 befindet, die Druckquelle 12 (Fig. 1) angeschlossen. Der aufgrund einer solchen Druckquelle resultierende Volumenstrom wird durch den unterhalb des Anschlussleitungselements 140 gezeigten und vertikal nach oben weisenden Pfeil, der die Pumpenflussrichtung kennzeichnet, dargestellt. Der durch die Druckquelle erzeugte Druck an der ersten Öffnung 136 reicht möglicherweise nicht aus, um den mittels des Ventilantriebs 34 erzeugten Druck in der Steuerdruckkammer 130 zu kompensieren. Entsprechend verschließt das Verschlusselement 134 die erste Öffnung 136 so lange, bis mittels des Ventilantriebs 34 in der Steuerdruckkammer 130 ein Steuerdruck erzeugt wird, dessen Kraft auf das Membranelement 132 kleiner ist als die Kraft, die aufgrund der Druckquelle auf das Membranelement 132 wirkt.

Die Druckkammer 138 weist weiter eine zweite Öffnung 142 auf, an welche ein zweites Anschlussleitungselement 144 anschließt. Das zweite Anschlussleitungselement 144 kann dabei mit weiteren pneumatischen Komponenten verbunden sein oder ein Auslass hin zu einem Patienten oder der Patientenschnittstelle 14 (Fig. 1)- bei einer Funktion als Inspirationsventil 26 - oder hin zu einer Umgebung - bei einer Funktion als Exspirationsventil 28 - sein. Solange das Verschlusselement 134 die erste Öffnung 136 verschließt, fließt kein Fluid durch die zweite Öffnung 142.

Die Darstellung in Figur 3b zeigt eine Situation, wie sie sich bei einem ausgeschalteten Ventilantrieb 34 ergibt. Der Ventilantrieb 34 (die Piezopumpe) bildet dabei eine offene fluidkommunizierende Verbindung zwischen der Steuerdruckkammer 130 und der Umgebung. D.h., dass zwischen der Steuerdruckkammer 130 und der Umgebung ein Druckausgleich stattfindet, sodass in der Steuerdruckkammer 130 Umgebungsdruck herrscht. Der Druck in dem ersten Anschlussleitungselement 140 ist - zum Beispiel aufgrund einer angeschlossenen Druckquelle 12 (Fig. 1) - nun größer als der Druck in der Steuerdruckkammer 130, der auf das Membranelement 132 wirkt. Daher wird das Membranelement 132 mit dem Verschlusselement 134 in die Steuerdruckkammer 130 hineingedrückt, sodass das Verschlusselement 134 die erste Öffnung 136 öffnet. Dann sind die erste Öffnung 136 und die zweite Öffnung 142 über die Druckkammer 138 fluidkommunizierend miteinander verbunden, sodass ein Fluid von der ersten Öffnung 136 zu der zweiten Öffnung 142 (und vom ersten Anschlussleitungselement 140 in das zweite Anschlussleitungselement 144) strömen kann. Der resultierende Volumenstrom ist durch den neben dem zweiten Anschlussleitungselement 144 gezeigten Pfeil veranschaulicht, welcher die Durchlassflussrichtung darstellt. Die pneumatische Ventilanordnung 30 ist nun geöffnet.

Die pneumatische Ventilanordnung 30 gemäß Figur 3 (Figuren 3a, 3b) und Figur 5 (Figuren 5a, 5b; Figuren 5c, 5d) kann als Proportionalventil fungieren. Je nachdem wie stark der Ventilantrieb 34 pumpt, d.h. wie groß der Druck in der Steuerdruckkammer 130 ist, kann der Abstand zwischen dem Verschlusselement 134 und der ersten Öffnung 136 gesteuert werden. Bei kleinen Abständen kann nur ein geringer Fluidstrom von der ersten Öffnung 136 zu der zweiten Öffnung 142 fließen. Bei großem Abstand, d.h. bei einem kleinen Steuerdruck, kann ein großer Fluidstrom zwischen der ersten Öffnung 136 und der zweiten Öffnung 142 fließen. Bei einer Funktion als Proportionalventil wird der Druckwiderstand an der ersten Öffnung 136 konstant gehalten.

Die Darstellungen in Figur 5 (Figuren 5a, 5b sowie Figuren 5c, 5d) zeigen eine spezielle Ausführungsform der Ventilanordnung 30 nach Figur 3. Diese Ventilanordnung 30 kann in dem Patientenmodul 20 (Fig. 2) als Inspirationsventil 26 eingesetzt werden und wird in Abhängigkeit vom Hinterdruck gesteuert. Dabei ist der Hinterdruck derjenige Druck, der sich bei dem aus der pneumatischen Ventilanordnung 30 herausfließenden Fluid einstellt. Dementsprechend ist der Vordruck der Druck, der sich beim Hineinfließen in die pneumatische Ventilanordnung 30 einstellt. Wenn - wie in Figur 5b dargestellt - bei geöffnetem Verschlusselement 134 das Fluid von der zweiten Öffnung 142 zu der ersten Öffnung 136 fließt, herrscht an der zweiten Öffnung 142 und der mit der zweiten Öffnung 142 verbundenen Druckkammer 138 der Vordruckzustand. An der ersten Öffnung 136 und dem damit verbundenen ersten Anschlussleitungselement 140 herrscht entsprechend der Hinterdruck.

Die Ausführungsformen gemäß Figur 5 umfassen zunächst die gleichen Elemente wie die Ausführungsform nach Figur 3, so dass auf die dortige Beschreibung verwiesen wird. Bezüglich der Strömungsrichtung durch die Ventilanordnung 30 gemäß Figur 5a, 5b ist eine im Vergleich zu der Strömungsrichtung durch die Ventilanordnung 30 gemäß Figur 3 umgekehrte Strömungsrichtung vorgesehen. Demgemäß kann an dem zweiten Anschlussleitungselement 144 eine Druckquelle, zum Beispiel eine Druckquelle 12 (Fig. 1), und an dem ersten Anschlussleitungselement 140 zumindest mittelbar eine Patientenschnittstelle 14 (Fig. 1) angeschlossen sein oder werden.

Zusätzlich zu der Ausführungsform gemäß Figur 3 umfasst die Ausführungsform gemäß Figur 5 eine zum Ventilantrieb 34 gehörende Verbindungskammer 146 und ein als Abzweig- oder Verbindungsleitung fungierendes Abzweigleitungselement 148. Aus der Verbindungskammer 146 wird beim Betrieb der Ventilanordnung 30 bei einem Pumpvorgang ein Fluid entnommen und mittels des Ventilantriebs (Piezopumpe) 34 in die Steuerdruckkammer 130 gepumpt.

Weiter ist die Verbindungskammer 146 fluidkommunizierend über das Abzweigleitungselement 148 mit dem ersten Anschlussleitungselement 140 verbunden. Über das Abzweigleitungselement 148 kann damit ein Druckausgleich zwischen dem ersten Anschlussleitungselement 140 sowie der ersten Öffnung 136 und der Verbindungskammer 146 stattfinden. In der Verbindungskammer 146 herrscht damit der Hinterdruck.

Wenn und solange der Ventilantrieb 34 eingeschaltet ist, herrscht in der Steuerdruckkammer 130 ein höherer Druck als in der Druckkammer 138 und an der ersten Öffnung 136. Daher wird das Membranelement 132 mit dem Verschlusselement 134 auf die erste Öffnung 136 gedrückt und verschließt die erste Öffnung 136. Ein Volumenstrom von der (eingangsseitigen) zweiten Öffnung 142 zur (ausgangsseitigen) ersten Öffnung 136 ist nicht möglich und ein eventueller vorheriger Volumenstrom wird unterbrochen,
Sobald der Ventilantrieb 34 ausgeschaltet ist, ergibt sich zwischen der Steuerdruckkammer 130 und der Verbindungskammer 146 eine offene fluidkommunizierende Verbindung (über den Zwei-Wege-Kanal 106; Fig. 3). Zwischen der Verbindungskammer 146 und der Steuerdruckkammer 130 kann damit ein Druckausgleich stattfinden, sodass sich in der Steuerdruckkammer 130 der Hinterdruck einstellt. Damit herrscht in der Steuerdruckkammer 130 der gleiche Druck wie an der ersten Öffnung 136 und wie am ersten Anschlussleitungselement 140.

Da der Vordruck in der Druckkammer 138 aufgrund der am zweiten Anschlussleitungselement 144 angeschlossenen Druckquelle größer ist als der Hinterdruck, wird das Membranelement 132 mit dem Verschlusselement 134 in die Steuerdruckkammer 130 hineingedrückt (von der ersten Öffnung 136 weg). Das Verschlusselement 134 wird damit in den Öffnungszustand versetzt, sodass die erste Öffnung 136 geöffnet ist. Damit kann zwischen der (eingangsseitigen) zweiten Öffnung 142 und der (ausgangsseitigen) ersten Öffnung 136 ein Fluid strömen. Bei einer Funktion als Inspirationsventil 26 in einem Patientenmodul 20 gemäß Figur 2 ist an das zur zweiten Öffnung 142 führende zweite Anschlussleitungselement 144 die Druckquelle 12 angeschlossen und das an die erste Öffnung 136 anschließende erste Anschlussleitungselement 140 ist zum Beispiel zum Innern des Patientenmoduls 20 und damit mittelbar zur Patientenschnittstelle 14 und den Atemwegen des Patienten offen oder an die Patientenschnittstelle 14 angeschlossen.

In Figur 5b ist ein Betriebszustand der Ventilanordnung 30 dargestellt, bei welchem der Ventilantrieb 34 einen Druck erzeugt, welcher gemeinsam mit dem Hinterdruck einen Druck in der Steuerdruckkammer 130 erzeugt, welcher bewirkt, dass das Membranelement 132 und das Verschlusselement 134 von der ersten Öffnung 136 weg ausgelenkt werden, so dass ein Volumenstrom von der zweiten Öffnung 142 hin zu der ersten Öffnung 136 möglich ist. Die Ansteuerung der Ventilanordnung 30 erfolgt also hinterdruckabhängig.

Die Ventilanordnung 30 gemäß Figur 5a, 5b stellt damit einen hinterdruckgesteuerten Druckwiderstand dar (die Ventilanordnung 30 ist ein hinterdruckgesteuerter Druckwiderstand / fungiert als hinterdruckgesteuerter Druckwiderstand). Beim Betrieb der Ventilanordnung 30 werden der Öffnungszustand des Verschlusselements 134 und der Abstand zwischen dem Verschlusselement 134 und der ersten Öffnung 136 abhängig vom Hinterdruck gesteuert. Je nach Größe des Hinterdrucks kann der Ventilantrieb (Piezopumpe) 34 nur ein bestimmtes Volumen in die Steuerdruckkammer 130 pumpen. Wenn ein geringer Hinterdruck herrscht, wird auch der Steuerdruck in der Steuerdruckkammer 130 geringer ausfallen als wenn ein höherer Hinterdruck herrschen würde. Bei einem geringeren Hinterdruck wird damit der Abstand zwischen dem Verschlusselement 134 und der ersten Öffnung 136 erhöht, da das Membranelement 132 durch den geringeren Steuerdruck, der aus dem geringeren Hinterdruck resultiert, tiefer in die Steuerdruckkammer 130 hineingedrückt wird als bei einem höheren Hinterdruck.

Die Darstellungen in Figur 5c, 5d zeigen eine weitere hinterdruckgesteuerte Ausführungsform der Ventilanordnung 30. Im Unterschied zu der Ausführungsform gemäß Figur 5a, 5b ist an diese Ventilanordnung 30 am ersten Anschlussleitungselement 140 eine Druckquelle, zum Beispiel eine Druckquelle 12 (Figur 1), und am zweiten Anschlussleitungselement 144 eine Patientenschnittstelle 14 (Fig. 1) angeschlossen oder anschließbar. Bei dieser Ausführungsform fungiert demnach das erste Anschlussleitungselement 140 als Eingang und das zweite Anschlussleitungselement 144 als Ausgang, wie dies auch durch die beiden Pfeile in der Darstellung in Figur 5d angedeutet ist. Bei der in Figur 5d gezeigten Situation ist ein Volumenstrom durch die Ventilanordnung 30 vom (eingangsseitigen) ersten Anschlussleitungselement 140 zum (ausgangsseitigen) zweiten Anschlussleitungselement 144 möglich und es erfolgt auch hierbei eine Hinterdrucksteuerung mittels des patientenseitigen (ausgangsseitigen) Drucks.

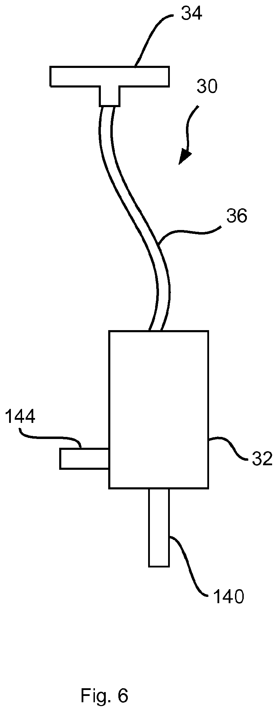

Für alle gezeigten Ventilanordnungen 30 (Figur 3, Figur 5) gilt, dass der Ventilantrieb 34 sich entweder - wie gezeigt - innerhalb des Gehäuses 32 der Ventilanordnung 30 befindet oder alternativ auch außerhalb des Gehäuses 32 und damit räumlich getrennt von den restlichen Komponenten der Ventilanordnung 30 platzierbar ist. Dann ist - wie dies die Darstellung in Figur 6 auf Basis der Darstellung in Figur 3a und in schematisch stark vereinfachter Form zeigt - der Ventilantrieb 34 mittels einer Verbindung in Form eines Schlauchs 36 oder dergleichen an das Gehäuse 32 der Ventilanordnung 30 angeschlossen und der Ventilantrieb 34 an die Steuerdruckkammer 130 angekoppelt. Auch dann ist der Ventilantrieb 34 zum Erzeugen eines Steuerdrucks in der Steuerdruckkammer 130 fluidkommunizierend mit der Steuerdruckkammer 130 verbunden. Für die Ausführungsformen gemäß Figur 5 gilt dies entsprechend, wobei der Ventilantrieb 34 dann in einem eigenen Gehäuse (nicht gezeigt) angeordnet ist, dessen Inneres als Verbindungskammer 146 fungiert, und wobei das Abzweigleitungselement 148 zum Beispiel in Form eines weiteren Schlauchs oder dergleichen einseitig in die Verbindungskammer 146 mündet.

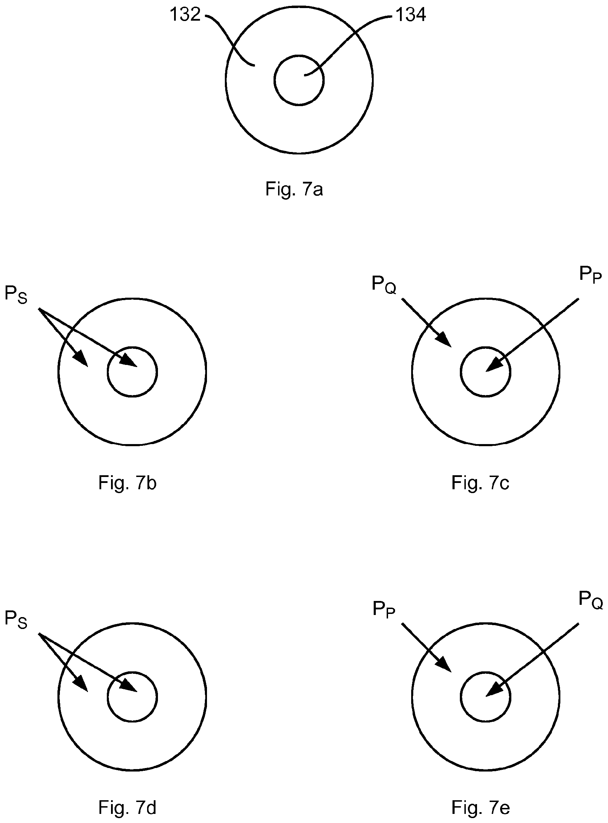

Figur 7 (Figuren 7a bis 7e) zeigt das Membranelement 132 und das Verschlusselement 134. Im Interesse einer besseren Übersicht sind die Bezugsziffern nur in der Darstellung in Figur 7a eingetragen. Das Membranelement 132 und das Verschlusselement 134 befinden sich im Innern des Gehäuses 32 der Ventilanordnung 30 und trennen die Steuerdruckkammer 130 von der Druckkammer 138.

Die Darstellung in Figur 7b zeigt die steuerdruckkammerseitige (Steuerdruckkammer 130) Oberfläche des Membranelements 132 (außen) und des Verschlusselements 134 (innen) der Ventilanordnung 30 in Figur 5a, 5b. Hier liegt sowohl in einem äußeren Bereich, also am Membranelement 132, wie auch in einem inneren Bereich, also am Verschlusselement 134, der Steuerdruck Ps aus der Steuerdruckkammer 130 an. Die Darstellung in Figur 7c zeigt die andere, also die druckkammerseitige (Druckkammer 138) Oberfläche des Membranelements 132 und des Verschlusselements 134 der Ventilanordnung 30 in Figur 5a, 5b. An dieser Seite der Oberfläche liegt im äußeren Bereich, also am Membranelement 132, der Druck der an das zweite Anschlussleitungselement 144 angeschlossenen Druckquelle P_{Q} und im inneren Bereich, also am Verschlusselement 134, der patientenseitige Druck P_{P} an.

Die Darstellung in Figur 7d zeigt die steuerdruckkammerseitige Oberfläche des Membranelements 132 und des Verschlusselements 134 der Ventilanordnung 30 in Figur 5c, 5d. Hier liegt sowohl in einem äußeren Bereich, also am Membranelement 132, wie auch in einem inneren Bereich, also am Verschlusselement 134, der Steuerdruck Ps aus der Steuerdruckkammer 130 an. Die Darstellung in Figur 7e zeigt die druckkammerseitige Oberfläche des Membranelements 132 und des Verschlusselements 134 der Ventilanordnung 30 in Figur 5c, 5d. An dieser Seite der Oberfläche liegt im äußeren Bereich, also am Membranelement 132, der patientenseitige Druck P_{P} und im inneren Bereich, also am Verschlusselement 134, der Druck der an das zweite Anschlussleitungselement 144 angeschlossenen Druckquelle P_{Q} an.

Bei einem Patientenmodul 20 gemäß Figur 2 umfasst dieses zwei Ventilanordnungen 30, nämlich eine erste, als Inspirationsventil 26 fungierende Ventilanordnung 30 und eine zweite, als Exspirationsventil 28 fungierende Ventilanordnung 30, wobei ein jeweils zugehöriger Ventilantrieb 34 entweder ebenfalls im Innern des Patientenmoduls 20 oder auch außerhalb des Patientenmoduls 20 angeordnet ist.

Bei einer als Inspirationsventil 26 fungierenden Ventilanordnung 30 gemäß Figur 3 ist der von der Druckquelle 12 kommende Inspirationsschlauch 16 an das erste Anschlussleitungselement 140 angeschlossen und das zweite Anschlussleitungselement 144 ist zum Innern des Patientenmoduls 20 offen oder an die Patientenschnittstelle 14 angeschlossen. Bei einer als Exspirationsventil 28 fungierenden Ventilanordnung 30 gemäß Figur 3 ist der zur Druckquelle 12 führende oder zur Umgebung hin offene Exspirationsschlauch 18 an das zweite Anschlussleitungselement 144 angeschlossen und das erste Anschlussleitungselement 140 ist zum Innern des Patientenmoduls 20 offen oder an die Patientenschnittstelle 14 angeschlossen.

Minimal umfasst das Patientenmodul 20 genau eine Ventilanordnung 30, nämlich eine als Exspirationsventil 28 fungierende Ventilanordnung 30, mit einem entweder im Innern des Patientenmoduls 20 oder außerhalb des Patientenmoduls 20 angeordneten Ventilantrieb 34.

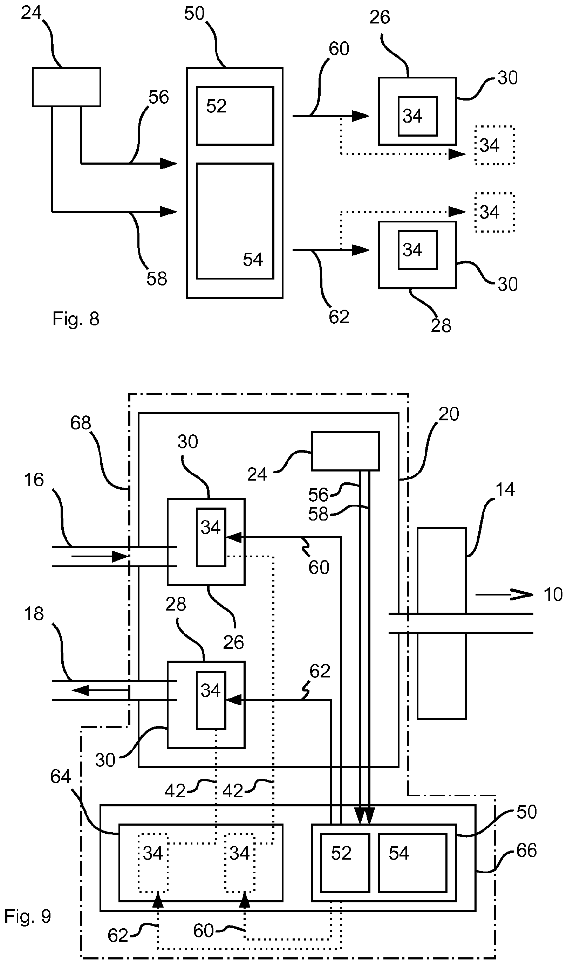

Die Darstellung in Figur 8 zeigt eine Steuerungseinrichtung 50, welche optional entweder Teil des Patientenmoduls 20 ist, genauso aber vom Patientenmodul 20 räumlich getrennt sein und zum Beispiel vom Patienten selbst getragen werden kann. Alternativ kommt auch in Betracht, dass die Steuerungseinrichtung 50 der Druckquelle 12 zugeordnet ist, also zum Beispiel Teil eines als Druckquelle 12 fungierenden Beatmungsgeräts oder dergleichen ist. Die Steuerungseinrichtung 50 umfasst in grundsätzlich an sich bekannter Art und Weise eine Verarbeitungseinheit 52 in Form von oder nach Art eines Mikroprozessors sowie einen Speicher, in den ein als Steuerungsprogramm 54 fungierendes Computerprogramm geladen ist.

Das Computerprogramm bestimmt die wesentliche Funktionalität des Patientenmoduls 20. Zur Veranschaulichung ist dazu in Figur 8 gezeigt, dass mittels der Steuerungseinrichtung 50 und unter Kontrolle des Steuerungsprogramms 54 von der Sensorik 24 erhältliche Sensorsignale 56, 58 zum Erhalt zumindest eines Steuersignals 60, 62 zur Ansteuerung des zumindest einen Ventils 28 (Exspirationsventil 28) oder der Ventile 26, 28 (Inspirationsventil 26, Exspirationsventil 28) - nämlich zur Ansteuerung des jeweiligen Ventilantriebs 34 - generierbar sind und beim Betrieb des Patientenmoduls 20 generiert werden. Als Sensorsignal 56, 58 oder Sensorsignale 56, 58 verarbeitet die Steuerungseinrichtung 50 zum Beispiel ein einen Druckmesswert kodierendes erstes Sensorsignal 56 und/oder ein einen Flowmesswert kodierendes zweites Sensorsignal 58. Die Ermittlung des zumindest einen Steuersignals 60, 62 erfolgt in grundsätzlich an sich bekannter Art und Weise zum Erhalt eines vorgegebenen oder vorgebbaren Druck- und/oder Volumenstromverlaufs während der Beatmung des Patienten mit zyklisch aufeinanderfolgenden inspiratorischen und exspiratorischen Phasen.

Bei einer nicht im Patientenmodul 20 angeordneten Steuerungseinrichtung 50 werden das oder die Sensorsignale 56, 58 leitungsgebunden oder leitungslos in grundsätzlich an sich bekannter Art und Weise an die Steuerungseinrichtung 50 übermittelt und das zumindest eine Steuersignal 60, 62 leitungsgebunden oder leitungslos an die jeweilige Ventilanordnung 30 und den davon umfassten Ventilantrieb 34 oder - bei einer Ausführungsform gemäß Figur 6 - an den jeweiligen Ventilantrieb 34 übermittelt. Die Auslagerung der Steuerungseinrichtung 50 aus dem Patientenmodul 20 hat den Vorteil, dass bei einer eventuell notwendigen Entsorgung des Patientenmoduls 20 die Steuerungseinrichtung 50 erhalten bleibt und mit einem anderen Patientenmodul 20 erneut verwendet werden kann. Wenn der Ventilantrieb 34 von der jeweiligen Ventilanordnung 30 räumlich getrennt ist (Figur 6), gilt dies ebenso für den oder jeden Ventilantrieb 34.

Die Ermittlung der Steuersignale 60, 62 und ein jeweiliger Druck- und/oder Volumenstromverlauf während der inspiratorischen und exspiratorischen Phasen steht bei der hier vorgestellten Neuerung nicht im Vordergrund, so dass insoweit auf den Stand der Technik verwiesen werden kann. Die Besonderheit besteht hier darin, dass einerseits die Sensorik 24 im Patientenmodul 20 oder jedenfalls nahe am Patientenmodul 20 angeordnet ist und dass andererseits die Ventile 26, 28 ebenfalls im Patientenmodul 20 angeordnet sind. Mittels der Sensorik 24 im Patientenmodul 20 oder nahe am Patientenmodul 20 - also jedenfalls nahe am Patienten - sind Messwerte, insbesondere Druck- und/oder Flowmesswerte, aufnehmbar, welche die tatsächlichen Verhältnisse in der Patientenlunge 10 besonders gut repräsentieren. Anders als bei einer zum Beispiel im Beatmungsgerät befindlichen Sensorik sind die mittels der Sensorik 24 im Patientenmodul 20 oder nahe am Patientenmodul 20 aufnehmbaren und im Betrieb aufgenommenen Messwerte nicht durch Laufzeiteffekte entlang des Schlauchsystems (Inspirationsschlauch 16 und/oder Exspirationsschlauch 18) zwischen Beatmungsgerät und Patientenschnittstelle 14 verfälscht. Auf diese Weise ist eine besonders exakte Steuerung oder Regelung des Druck- und/oder Volumenstromverlaufs während der inspiratorischen und exspiratorischen Phasen möglich.

Das Patientenmodul 20 ist modulartig in den Atemgasweg zwischen Druckquelle 12 und Patientenlunge 10 eingebunden oder in diesem Atemgasweg einbindbar. Dazu weist das Patientenmodul 20 auf einer der Druckquelle 12 zugewandten Eingangsseite zumindest eine genormte Anschlussstelle und auf einer dem Patienten zugewandten Ausgangsseite ebenfalls zumindest eine genormte Anschlussstelle auf. Entsprechend erfolgt der Anschluss eines Inspirationsschlauchs 16 oder eines Inspirationsschlauchs 16 und eines Exspirationsschlauchs 18 an das Patientenmodul 20 bevorzugt mittels genormter Anschlussstellen in Form von jeweils durch einen sogenannten Medizinkonus gebildeten Anschlussstellen. Im Innern des Patientenmoduls 20 sind die jeweiligen Ventilanordnungen 30 oder die zumindest eine Ventilanordnung 30 fluidkommunizierend an diese Anschlussstellen angeschlossen.

Die Patientenschnittstelle 14 ist bevorzugt ebenfalls mittels zumindest einer derartigen genormten Anschlussstelle in Form von zumindest einem als Anschlussstelle fungierenden Medizinkonus mit dem Patientenmodul 20 lösbar verbindbar. Durch Lösen der an die jeweiligen Anschlussstellen angeschlossenen Einheiten kann ein Patientenmodul 20 leicht ausgetauscht und bei Bedarf durch ein anderes Patientenmodul 20 ersetzt werden.

Die Darstellung in Figur 9 zeigt das Patientenmodul 20 gemäß Figur 2 mit weiteren Einzelheiten. Für jede Ventilanordnung 30 ist jeweils ein Ventilantrieb 34 mit einer Umrandung mit durchgezogenen Linien sowie mit einer gestrichelten Umrandung dargestellt. Dies soll veranschaulichen, dass der Ventilantrieb 34 der jeweiligen Ventilanordnung 30 räumlich zugeordnet sein kann (Umrandung mit durchgezogenen Linien) oder auch räumlich entfernt von der jeweiligen Ventilanordnung 30 angeordnet sein kann (Umrandung mit gestrichelten Linien). Jeder Ventilantrieb 34 kann sich in einem eigenen Gehäuse befinden. Der Ventilantrieb 34 bildet zusammen mit seinem Gehäuse dann ein Ventilantriebsmodul 64. Zwei von dem Patientenmodul 20 räumlich getrennte Ventilantriebe 34 können auch zusammen in einem Gehäuse angeordnet sein und ebenfalls ein Ventilantriebsmodul 64 bilden.

Grundsätzlich kommen für das hier vorgeschlagene Patientenmodul 20 hinsichtlich des Orts des oder jedes Ventilantriebs 34 alle möglichen Permutationen in Betracht. Es war bereits erläutert worden, dass das Patientenmodul 20 zumindest eine Ventilanordnung 30 umfasst, insbesondere eine als Exspirationsventil 28 fungierende Ventilanordnung 30. Der Ventilantrieb 34 dieser zumindest einen Ventilanordnung 30 kann sich innerhalb des Patientenmoduls 20 oder räumlich entfernt von dem Patientenmodul 20 außerhalb des Patientenmoduls 20 befinden, zum Beispiel in einem Ventilantriebsmodul 64. Bei zumindest zwei von dem Patientenmodul 20 umfassten Ventilanordnungen 30 kann sich jeder Ventilantrieb 34 der zumindest zwei Ventilanordnungen 30 innerhalb oder außerhalb des Patientenmoduls 20 befinden. Bei jedem außerhalb des Patientenmoduls 20 befindlichen Ventilantrieb 34, insbesondere einem in einem außerhalb des Patientenmoduls 20 in einem Gehäuse eines Ventilantriebsmoduls 64 angeordneten Ventilantrieb 34, ist dieser pneumatisch mit dem im Patientenmodul 20 befindlichen Teil der jeweiligen Ventilanordnung 30 verbunden, insbesondere in einer Art und Weise, wie dies weiter oben im Zusammenhang mit der Erläuterung der Darstellung in Figur 6 ausgeführt wurde.

Bei einem externen Ventilantrieb 34 der zumindest einen Ventilanordnung 30 (Exspirationsventil 28) oder externen Antrieben 34 des Inspirationsventils 26 und/oder des Exspirationsventils 28 ist bevorzugt auch der oder jeder Ventilantrieb 34 lösbar mit dem Patientenmodul 20 verbindbar und beim Betrieb des Patientenmoduls 20 lösbar mit diesem verbunden. Die Verbindung besteht in Form des Schlauchs 36 (Fig. 6). Nicht gezeigte Adern (elektrische Verbindungsleitungen) zum Anlegen einer elektrischen Spannung an das Piezoelement 118 (Fig. 4) des Ventilantriebs 34 verlaufen entweder ebenfalls zum Patientenmodul 20 oder zu einer weiteren Einheit, zum Beispiel der Steuerungseinrichtung 50.

Zum Trennen eines Ventilantriebs 34 vom Patientenmodul 20 werden der Schlauch 36 und ggf. diese Adern gelöst. Die Adern sind zum Beispiel auf einen Stecker geführt, der im Innern des Patientenmoduls 20 (oder alternativ im Innern einer vom Patientenmodul 20 räumlich getrennten Steuerungseinrichtung 50) in eine dort vorgesehene Anschlussbuchse einsteckbar ist. Die Adern sind damit lösbar mit dem Patientenmodul 20 und/oder mit dem Ventilantriebsmodul 64 verbindbar. Der Schlauch 36 zum Ventilantrieb 34 ist an zumindest einer Verbindungsstelle lösbar mit dem Ventilantrieb 34 verbunden und damit insgesamt ebenfalls lösbar mit dem Patientenmodul 20 verbunden. Durch Lösen der Adern und des Schlauchs 36 kann jeder Ventilantrieb 34 vom Patientenmodul 20 getrennt werden. Die Adern und der Schlauch 36 können auf einen gemeinsamen Stecker und eine dazu passende Anschlussbuchse geführt sein.

Bei einer externen Steuerungseinrichtung 50 mit Adern für die leitungsgebundene Übertragung des zumindest einen Sensorsignals 56, 58 und des zumindest einen Steuersignals 60, 62 gilt das oben in Bezug auf die zu einem Ventilantrieb 34 führenden Adern Gesagte entsprechend. Auch hier ist zur lösbaren Verbindung der Steuerungseinrichtung 50 mit dem Patientenmodul 20 vorgesehen, dass die entsprechenden Adern zumindest einseitig auf einen Stecker oder dergleichen geführt sind, die im Patientenmodul 20 oder auf Seiten der Steuerungseinrichtung 50 in eine entsprechende Anschlussbuchse einsteckbar sind. Durch Lösen der Adern kann eine von dem Patientenmodul 20 räumlich entfernte Steuerungseinrichtung 50 vom Patientenmodul 20 getrennt werden.

Bei einer vom Patientenmodul 20 räumlich entfernten Steuerungseinrichtung 50 sowie zumindest einem vom Patientenmodul 20 räumlich entfernten Ventilantrieb 34 können die Steuerungseinrichtung 50 sowie der oder jeder Ventilantrieb 34, insbesondere in Form eines den oder jeden Ventilantrieb 34 umfassenden Ventilantriebsmoduls 64, in einem im Folgenden als Steuerungsmodul 66 bezeichneten Geräteteil zusammengefasst sein. Dieses kann der Patient tragen, zum Beispiel um den Hals. Im Falle einer notwendigen Entsorgung des Patientenmoduls 20 bleibt das Steuerungsmodul 66 erhalten und kann weiter verwendet werden. Bei einem notwendigen Austausch des Patientenmoduls 20, zum Beispiel zu Reinigungszwecken, kann das auszutauschende Patientenmodul 20 schnell und unkompliziert durch ein neues Patientenmodul 20 oder ein aufbereitetes Patientenmodul 20 ausgetauscht werden.

Insgesamt ergibt sich ein Patientenmodulsystem 68. Der Umfang des Patientenmodulsystems 68 ist vom Ort des zumindest einen Ventilantriebs 34 oder der Ventilantriebe 34 und/oder vom Ort der Steuerungseinrichtung 50 abhängig. Danach umfasst das Patientenmodulsystem 68 zumindest das Patientenmodul 20. Je nach Ausführung umfasst das Patientenmodulsystem 68 einen externen Ventilantrieb 34 oder zwei externe Ventilantriebe 34, wobei der oder jeder Ventilantrieb 34 in einem Ventilantriebsmodul 64 angeordnet sein kann und das Patientenmodulsystem 68 dann auch ein solches Ventilantriebsmodul 64 umfasst. Weiter umfasst das Patientenmodulsystem 68 ggf. eine externe Steuerungseinrichtung 50. Wenn die externe Steuerungseinrichtung 50 und der oder jeder externe Ventilantrieb 34 oder ein den oder jeden externen Ventilantrieb 34 umfassendes Ventilantriebsmodul 64 zu einem Steuerungsmodul 66 zusammengefasst sind, umfasst das Patientenmodulsystem 68 auch ein solches Steuerungsmodul 66.

Einzelne im Vordergrund stehende Aspekte der hier eingereichten Beschreibung lassen sich damit kurz wie folgt zusammenfassen: Angegeben wird eine zur Verwendung zusammen mit einer Druckquelle 12 vorgesehene und hier als Patientenmodul 20 bezeichnete Vorrichtung zum Beatmen eines Patienten. Diese zeichnet sich dadurch aus, dass sie die Druckquelle 12 strömungsmäßig an eine mit den Atemwegen eines Patienten verbindbare Patientenschnittstelle 14 koppelt und dass sie zumindest eine Ventilanordnung 30 umfasst, welche mittels einer als Ventilantrieb 34 fungierenden, vorzugsweise hochfrequent betreibbaren Piezopumpe steuerbar ist, und wobei die zumindest eine Ventilanordnung 30 als Exspirationsventil 28 fungiert.

### BEZUGSZEICHENLISTE

- 10: Patientenlunge
- 12: Druckquelle
- 14: Patientenschnittstelle
- 16: Beatmungsschlauch, Inspirationsschlauch
- 18: Beatmungsschlauch, Exspirationsschlauch
- 20: Patientenmodul
- 22: Tubus
- 24: Sensorik
- 26: Ventil, Inspirationsventil
- 28: Ventil, Exspirationsventil
- 30: Ventilanordnung
- 32: Gehäuse
- 34: Antrieb, Ventilantrieb
- 36: Schlauch

- 102: erste Zwei-Wege-Durchlassöffnung
- 104: zweite Zwei-Wege-Durchlassöffnung
- 106: Zwei-Wege-Kanal
- 108: Außengehäuse
- 110: Innengehäuse
- 112: Abdeckplatte
- 114: Pumpöffnung
- 116: Pumpenkammer
- 118: Piezoelement
- 120: Pumpenmembranelement
- 122: Verbindungselement
- 124: Wechselspannungsgenerator

- 130: Steuerdruckkammer
- 132: Membranelement
- 134: Verschlusselement
- 136: erste Öffnung
- 138: Druckkammer
- 140: erstes Anschlussleitungselement
- 142: zweite Öffnung
- 144: zweites Anschlussleitungselement
- 146: Verbindungskammer
- 148: Abzweigleitungselement

- 50: Steuerungseinrichtung
- 52: Verarbeitungseinheit
- 54: Steuerungsprogramm
- 56: Sensorsignal
- 58: Sensorsignal
- 60: Steuersignal
- 62: Steuersignal
- 64: Ventilantriebsmodul
- 66: Steuerungsmodul
- 68: Patientenmodulsystem

## Patentansprüche

1. Patientenmodul (20) zur Verwendung zusammen mit einer Druckquelle (12), wobei das Patientenmodul (20) die Druckquelle (12) strömungsmäßig an eine mit den Atemwegen eines Patienten verbindbare Patientenschnittstelle (14) koppelt und das Patientenmodul (20) lösbar mit der Patientenschnittstelle (14) verbindbar ist,
wobei das Patientenmodul (20) zumindest zwei Ventilanordnungen (30) mit jeweils außerhalb des Patientenmoduls (20) anordenbaren Ventilantrieben (34), umfasst wobei eine der zumindest zwei Ventilanordnungen (30) als Exspirationsventil (28) und eine der Ventilanordnungen (30) als Inspirationsventil (26) fungiert,
wobei jede Ventilanordnung (30) einen Ventilantrieb (34), eine Druckkammer (138) und eine Steuerdruckkammer (130) umfasst und der Ventilantrieb (34) zum Erzeugen eines Steuerdrucks in der Steuerdruckkammer (130) fluidkommunizierend mit der Steuerdruckkammer (130) verbunden ist,
wobei die Steuerdruckkammer (130) von der Druckkammer (138) mittels eines ein Verschlusselement (134) aufweisenden Membranelements (132) getrennt ist,
wobei mittels des Verschlusselements (134) eine erste Öffnung (136) der Druckkammer (138) geöffnet und verschlossen werden kann und das Verschlusselement (134) über das Membranelement (132) mittels des Steuerdrucks steuerbar ist,
wobei als Ventilantrieb (34) eine Piezopumpe fungiert,
wobei
in jeder, als Inspirationsventil (26) fungierenden Ventilanordnung (30) ein Abzweigleitungselement (148) eine Verbindungskammer (146) fluidkommunizierend mit genau einem von zumindest zwei in der Druckkammer (138) mündenden Anschlussleitungselementen (140, 144) verbindet und
wobei der Ventilantrieb (34) so angeordnet ist, dass er Gas von der Verbindungskammer (146) in die Steuerdruckkammer (130) pumpt bzw. dass er eine Gasförderung von der Verbindungskammer (146) in die Steuerdruckkammer (130) derart vornimmt, dass er eine Druckerhöhung in der Steuerdruckkammer bewirkt.

2. Patientenmodul (20) nach Anspruch 1,
wobei der oder jeder Ventilantrieb (34) ein mit einer elektrischen Spannung beaufschlagbares Piezoelement (118) umfasst und wobei mittels des Piezoelements (118) durch eine spannungsabhängige Formänderung des Piezoelements (118) ein Pumpenmembranelement (120) des Ventilantriebs (34) beweglich ist.

3. Patientenmodul (20) nach Anspruch 1 oder 2,
wobei jeder Ventilantrieb (34) außerhalb des Patientenmoduls (20) fluidkommunizierend mit jeweils einer Ventilanordnung (30) verbunden oder verbindbar ist.

4. Patientenmodul (20) nach einem der vorangehenden Ansprüche,
mit zumindest zwei in der Druckkammer (138) mündenden Anschlussleitungselementen (140, 144),
wobei an eines der zumindest zwei in der Druckkammer (138) mündenden Anschlussleitungselemente (140) die Patientenschnittstelle (14) und an das andere Anschlussleitungselement (144) die Druckquelle (12) anschließbar ist.

5. Patientenmodul (20) nach einem der vorangehenden Ansprüche,
mit Mitteln zum lösbaren Verbinden mit zumindest einem von der Druckquelle (12) kommenden Beatmungsschlauch (16, 18).

6. Patientenmodulsystem (68) mit einem Patientenmodul (20) nach einem der vorangehenden Ansprüche sowie einer innerhalb oder außerhalb des Patientenmoduls (20) anordenbaren Steuerungseinrichtung (50),
wobei das Patientenmodul (20) ferner eine Sensorik (24) umfasst,
wobei mittels der Steuerungseinrichtung (50) auf Basis zumindest eines von der Sensorik (24) erhältlichen Sensorsignals (56, 58) zumindest ein Steuersignal (60, 62) zur Ansteuerung zumindest einer Ventilanordnung (30) des Patientenmoduls (20) generierbar ist.

7. Patientenmodulsystem (68) nach Anspruch 6,
wobei die Steuerungseinrichtung (50) vom Patientenmodul (20) räumlich getrennt und zum Erhalt des zumindest einen Sensorsignals (56, 58) von der Sensorik (24) sowie zur Übermittlung des zumindest einen Steuersignals (60, 62) an zumindest eine Ventilanordnung (30) des Patientenmoduls (20) mit dem Patientenmodul (20) kommunikativ verbunden ist.

8. System mit einem Patientenmodul (20) nach einem der Ansprüche 1 bis 5 oder mit einem Patientenmodulsystem (68) nach Anspruch 6 oder 7,
wobei als Druckquelle (12) ein Beatmungsgerät fungiert und das Beatmungsgerät als Bedien- und Benutzerschnittstelle für das Patientenmodul (20) fungiert.

9. System mit einem Patientenmodulsystem (68) nach Anspruch 6 oder 7,
wobei als Druckquelle (12) ein Beatmungsgerät fungiert und wobei das Beatmungsgerät die Steuerungseinrichtung (50) aufweist und als Bedien- und Benutzerschnittstelle für das Patientenmodul (20) fungiert.

10. System mit einem Patientenmodul (20) nach einem der Ansprüche 1 bis 5 oder mit einem Patientenmodulsystem (68) nach Anspruch 6 oder 7,
wobei als Druckquelle (12) eine Konstantdruckquelle fungiert und insbesondere die Steuerungseinrichtung (50) als Bedien- und Benutzerschnittstelle für das Patientenmodul (20) fungiert.

## Claims

1. Patient module (20) for use together with a pressure source (12),
wherein the patient module (20) in terms of flow couples the pressure source (12) to a patient interface (14) that is connectable to the airways of a patient and the patient module (20) is detachably connectable to the patient interface (14),
wherein the patient module (20) comprises at least two valve arrangements (30) with valve drives (34) that are arrangeable outside of the patient module (20) in each case, wherein one of the at least two valve arrangements (30) acts as an expiration valve (28) and one of the valve arrangements (30) acts as an inspiration valve (26),
wherein each valve arrangement (30) comprises a valve drive (34), a pressure chamber (138) and a control pressure chamber (130), and the valve drive (34) is fluid-communicatively connected to the control pressure chamber (130) for the purposes of producing a control pressure in the control pressure chamber (130),
wherein the control pressure chamber (130) is separated from the pressure chamber (138) by means of a membrane element (132) having a closure element (134),
wherein the closure element (134) can be used to open and close a first opening (136) in the pressure chamber (138) and the closure element (134) is controllable by means of the control pressure via the membrane element (132),
wherein a piezo-pump acts as a valve drive (34), wherein, in each valve arrangement (30) acting as an inspiration valve (26), a branch conduit element (148) fluid-communicatively connects a connecting chamber (146) to exactly one of at least two connection conduit elements (140, 144) that open up in the pressure chamber (138) and
wherein the valve drive (34) is arranged such that it pumps gas from the connecting chamber (146) into the control pressure chamber (130) or such that it conveys gas from the connecting chamber (146) into the control pressure chamber (130) in such a way that it brings about a pressure increase in the control pressure chamber.

2. Patient module (20) according to Claim 1,
wherein the, or each, valve drive (34) comprises a piezo element (118) to which an electric voltage is able to be applied and wherein a pump membrane element (120) of the valve drive (34) is movable by means of the piezo element (118) as a result of a voltage-dependent shape change of said piezo element (118) .

3. Patient module (20) according to Claim 1 or 2,
wherein each valve drive (34) is fluid-communicatively connected or connectable outside of the patient module (20) to a respective valve arrangement (30).

4. Patient module (20) according to any one of the preceding claims,
comprising at least two connection conduit elements (140, 144) that open up in the pressure chamber (138),
wherein the patient interface (14) is connectable to one of the at least two connection conduit elements (140) that open up in the pressure chamber (138) and the pressure source (12) is connectable to the other connection conduit element (144).

5. Patient module (20) according to any one of the preceding claims, comprising means for the detachable connection to at least one ventilation tube (16, 18) coming from the pressure source (12).

6. Patient module system (68) comprising a patient module (20) according to any one of the preceding claims and a control device (50) that is arrangeable within or outside of the patient module (20), wherein the patient module (20) further comprises a sensor system (24),
wherein at least one control signal (60, 62) for driving at least one valve arrangement (30) of the patient module (20) is generable by means of the control device (50) on the basis of at least one sensor signal (56, 58) obtainable from the sensor system (24).

7. Patient module system (68) according to Claim 6,
wherein the control device (50) is spatially separated from the patient module (20) and communicatively connected to the patient module (20) for receiving the at least one sensor signal (56, 58) from the sensor system (24) and for transmitting the at least one control signal (60, 62) to at least one valve arrangement (30) of the patient module (20) .

8. System comprising a patient module (20) according to any one of Claims 1 to 5 or comprising a patient module system (68) according to Claim 6 or 7, wherein a ventilator acts as pressure source (12) and the ventilator acts as operating and user interface for the patient module (20).

9. System comprising a patient module system (68) according to Claim 6 or 7,
wherein a ventilator acts as pressure source (12) and wherein the ventilator comprises the control device (50) and acts as operating and user interface for the patient module (20).

10. System comprising a patient module (20) according to any one of Claims 1 to 5 or comprising a patient module system (68) according to Claim 6 or 7, wherein a constant pressure source acts as pressure source (12) and, in particular, the control device (50) acts as operating and user interface for the patient module (20).

## Revendications

1. Module (20) pour patient, conçu pour être utilisé conjointement à une source de pression (12),
sachant que ledit module (20) pour patient instaure une liaison d'écoulement de ladite source de pression (12) avec un interface (14) de patient pouvant être raccordé aux voies respiratoires d'un patient, et que ledit module (20) pour patient peut être raccordé amoviblement audit interface (14) du patient,
lequel module (20) pour patient inclut au moins deux ensembles de distribution (30) comportant des entraînements (34) de valves pouvant être respectivement disposés à l'extérieur dudit module (20) pour patient, l'un des deux ensembles de distribution (30) à présence minimale remplissant la fonction d'une valve expiratoire (28), et l'un desdits ensembles de distribution (30) agissant comme une valve inspiratoire (26),
chaque ensemble de distribution (30) incluant un entraînement (34) de valve, une chambre de pression (138) et une chambre (130) de pression de commande, et ledit entraînement (34) de valve étant en communication fluidique avec la chambre (130) de pression de commande, en vue d'engendrer une pression de commande dans ladite chambre (130) de pression de commande,
laquelle chambre (130) de pression de commande est séparée d'avec ladite chambre de pression (138) au moyen d'un élément (132) à membrane, muni d'un élément obturateur (134),
un premier orifice (136) de ladite chambre de pression (138) pouvant être ouvert et fermé au moyen dudit élément obturateur (134), et ledit élément obturateur (134) pouvant être piloté au moyen de la pression de commande, par l'intermédiaire de l'élément (132) à membrane,
une pompe piézoélectrique remplissant la fonction d'entraînement (34) de valve, sachant que, dans chaque ensemble de distribution (30) agissant comme une valve inspiratoire (26), un élément de conduit de bifurcation (148) établit une communication fluidique d'une chambre de jonction (146) avec précisément l'un d'au moins deux éléments de conduits de raccordement (140, 144) débouchant dans la chambre de pression (138), et
sachant que ledit entraînement (34) de valve est agencé de telle sorte qu'il pompe, dans la chambre (130) de pression de commande, du gaz provenant de la chambre de jonction (146), respectivement de telle sorte qu'il gouverne un afflux de gaz dans ladite chambre (130) de pression de commande, à partir de ladite chambre de jonction (146), de manière qu'il provoque un accroissement de pression dans ladite chambre de pression de commande.

2. Module (20) pour patient, selon la revendication 1,
sachant que l'entraînement, ou chaque entraînement (34) de valve inclut un élément piézoélectrique (118) pouvant être sollicité par une tension électrique, et sachant qu'un élément (120) à membrane de pompage dudit entraînement (34) de valve est mobile au moyen de l'élément piézoélectrique (118), par une modification de la forme dudit élément piézoélectrique (118) dépendante de la tension.

3. Module (20) pour patient, selon la revendication 1 ou 2,
sachant que chaque entraînement (34) de valve est, ou peut être raccordé à un ensemble respectif de distribution (30), avec communication fluidique, à l'extérieur dudit module (20) pour patient.

4. Module (20) pour patient, selon l'une des revendications précédentes,
muni d'au moins deux éléments de conduits de raccordement (140, 144) débouchant dans la chambre de pression (138),
sachant que l'interface (14) du patient et la source de pression (12) peuvent être raccordés, respectivement, à l'un (140) des deux éléments de conduits de raccordement à présence minimale, débouchant dans ladite chambre de pression (138), et à l'autre élément de conduit de raccordement (144).

5. Module (20) pour patient, selon l'une des revendications précédentes,
doté de moyens dévolus à la liaison amovible avec au moins un tuyau respiratoire (16, 18) émanant de la source de pression (12).

6. Système (68) à module pour patient, comprenant un module (20) pour patient conforme à l'une des revendications précédentes, ainsi qu'un dispositif de commande (50) pouvant être placé à l'intérieur ou à l'extérieur dudit module (20) pour patient,
ledit module (20) pour patient incluant, par ailleurs, un ensemble de détection (24),
sachant qu'au moins un signal de commande (60, 62) peut être engendré au moyen du dispositif de commande (50), en vue du pilotage d'au moins un ensemble de distribution (30) dudit module (20) pour patient, sur la base d'au moins un signal de détection (56, 58) pouvant être procuré par ledit ensemble de détection (24).

7. Système (68) à module pour patient, selon la revendication 6,
sachant que le dispositif de commande (50) est spatialement séparé d'avec le module (20) pour patient et est en liaison communicante avec ledit module (20) pour patient, en vue d'acquérir le signal de détection (56, 58) à présence minimale, provenant de l'ensemble de détection (24), ainsi que de transmettre le signal de commande (60, 62), à présence minimale, à au moins un ensemble de distribution (30) dudit module (20) pour patient.

8. Système équipé d'un module (20) pour patient conforme à l'une des revendications 1 à 5, ou d'un système (68) à module pour patient conforme à la revendication 6 ou 7,
sachant qu'un appareil respiratoire remplit la fonction de source de pression (12), et que ledit appareil respiratoire agit en tant qu'interface d'actionnement et d'utilisation affecté audit module (20) pour patient.

9. Système équipé d'un système (68) à module pour patient conforme à la revendication 6 ou 7,
sachant qu'un appareil respiratoire remplit la fonction de source de pression (12), et que ledit appareil respiratoire est muni du dispositif de commande (50) et agit en tant qu'interface d'actionnement et d'utilisation affecté audit module (20) pour patient.

10. Système équipé d'un module (20) pour patient conforme à l'une des revendications 1 à 5, ou d'un système (68) à module pour patient conforme à la revendication 6 ou 7,
sachant qu'une source de pression constante remplit la fonction de source de pression (12) et que, notamment, le dispositif de commande (50) agit en tant qu'interface d'actionnement et d'utilisation affecté audit module (20) pour patient.
